# EUROPEAN PATENT SPECIFICATION

(11) **EP 0 612 744 B1**
(45) Date of publication and mention of the grant of the patent: **23.12.1998**
(21) Application number: 93200524.2
(22) Date of filing: 24.02.1993
(51) Int. Cl.: C07D 473/12, A23F 5/22

(54) **Regeneration of caffeine laden active carbon, using organic solvents**
Wiedergewinnung von mit Kaffein beladener Aktivkohle unter Verwendung von organischen Solventen
Régénération simultanée de la caféine et charbon actif auquel elle était adsorbée au moyen de solvants organiques

(43) Date of publication of application: 31.08.1994
(73) Proprietor: Sara Lee/DE N.V., 3532 AA Utrecht (NL)
(72) Inventor: Noomen, Pieter Jan, NL-3572 ZA Utrecht (NL); van Putten, Corstiaan, NL-4101 BC Culemborg (NL)
(74) Representative: Smulders, Theodorus A.H.J., Ir.

(56) References cited:
- EP-A- 0 042 295
- EP-A- 0 076 620
- EP-A- 0 129 609
- EP-A- 0 129 610
- EP-A- 0 248 482
- EP-A- 0 251 364
- EP-A- 0 259 905
- US-A- 4 443 601
- CHEMICAL ABSTRACTS, vol. 50, no. 9, 10 May 1956, Columbus, Ohio, US; abstract no. 6101f, page 6101 ;column L ;
- CHEMICAL ABSTRACTS, vol. 48, no. 16, 25 August 1954, Columbus, Ohio, US; abstract no. 9151e, page 9151 ;column L ;

## Description

The present invention is directed to a process for recovering caffeine from activated carbon loaded with caffeine, as well as to a process for decaffeinating green coffee.

The decaffeination of vegetable materials is a commercially important process, especially with respect to the decaffeination of green coffee, but also tea and other vegetable materials can be decaffeinated with advantage to obtain caffeine-free or low caffeine materials.

In the art numerous processes have been described for decaffeination of vegetable materials. A number of said processes is based upon the direct extraction of caffeine from the materials, for example with solvents or supercritical carbon dioxide. However, the most important processes are based upon the principle of extracting at least the caffeine from the vegetable material into an aqueous liquid, from which aqueous liquid the caffeine is recovered and the aqueous liquid is re-used.

The recovery of the caffeine from the aqueous liquid can be done in a number of ways. The present invention is directed with an improvement of one of the methods used therefor, namely the method of recovering the caffeine from the aqueous liquid using activated carbon. The present invention more in particular deals with an improvement in the regeneration of the activated carbon in order to carry out the regeneration in an improved way, resulting in a process that is more economically attractive.

In the art various methods have been disclosed for carbon caffeine separation. For example in the European patent application 42,295 a process for recovering caffeine from activated carbon is disclosed, said process comprising contacting activated carbon having caffeine adsorbed thereon with a caffeine solvent comprising an organic acid or an alcohol. More in particular in the examples glacial acetic acid or mixtures thereof with other organic chemicals have been used. In the European patent application 76620 of the same applicant as the above-identified European patent application dilute solutions of acetic acids have been disclosed.

In the European patent applications 248,482, 251,362 and 259,905 various organic acids and mixtures thereof have been proposed for desorption of caffeine from activated carbon. In the European patent applications 129,609 and 129,610 the use of water and an aqueous formic acid solution have been disclosed.

Although a number of the above proposals have led to an improved recovery of the caffeine from the activated carbon there is still a need for further improvement, both in terms of energy requirements and in terms of speed of extraction.

The present invention is based upon the surprising discovery that a number of well-known and relatively simple chemical compounds possess superior recovery properties in terms of speed of recovery and energy requirements.

The invention is directed to a process for recovering caffeine from activated carbon loaded with caffeine, said process being characterized in that said activated carbon having caffeine adsorbed thereon is contacted with methylethylketone, ethyl acetate, dichloromethane or a mixture of methylethylketone and ethyl acetate.

Surprisingly it has been found that the use of the various recovery agents disclosed herein results in a much more economical recovery of caffeine from activated carbon than glacial acetic acid, which was up to now considered to be a very efficient recovery agent. In terms of loss of dry solids during the regeneration, that is the non-caffeine soluble solids adsorbed on the activated carbon that are extracted by the recovery agent, and/or in terms of energy requirements it turns out that overall the use of acetic acid is much less economical. If one compares the total economical situation of the recovery agents according to the present invention in relation to glacial acetic acid or diluted acetic acid it turns out that acetic acid is 50 to 200% less economical than the recovery agents according to the present invention. Ethanol, also a known recovery agent, has been found to be even more uneconomical than acetic acid.

Apart from the use of the specific recovery agents the processes of the present invention are carried out in a manner known in the art.

The activated carbon is contacted with the recovery agent, whereby the caffeine is desorbed from the carbon and becomes dissolved in the recovery agent. The various process conditions, such as time, temperature, pressure, ratio carbon/recovery agent and the like, can be easily determined on the basis of some routine experiments. These conditions may of course vary depending on the type of recovery agent used.

Suitable temperatures range from about 25°C to above 100°C. Temperatures above 150°C do not give additional advantages. The choice of the recovery agent in combination with the temperature determines the minimum pressure to be used in accordance with the invention. In case the temperature is at or below the boiling point of the recovery agent ambient pressure can be used. In case the temperature is above the boiling point, an increased pressure is necessary. Generally the pressure used in the recovery ranges from 1 bar to about 10 bar.

The ratio of activated carbon to recovery agent should preferably at least be sufficient to recover so much caffeine from the carbon that it may be re-used to remove caffein from extracts. This depends in part on the activity of the recovery agent, but also on the solubility of caffeine in the recovery agent. Preferably the amount of recovery agent in relation to the activated carbon is calculated on the basis of the amount of caffeine on the carbon. Suitable amounts range from 5 to 250 parts by weight of recovery agent per part of weight of caffeine present on the carbon.

The invention is also directed to a process for decaffeinating green coffee, said process comprising extracting at least the caffeine from the green coffee into an aqueous liquid, adsorbing the caffeine from the said aqueous liquid on activated carbon and contacting said activated carbon having caffeine adsorbed thereon with methylethylketone, ethyl acetate, dichloromethane or a mixture of methylethylketone and ethyl acetate to remove the caffeine from the activated carbon.

For the decaffeination of green coffee various well-known methods can be used, either based upon preparing an aqueous extract of substantially all soluble coffee solids including caffeine, extracting the caffeine from the aqueous extract using activated carbon and returning the remainder of the extract to the green coffee, or a method where the green coffee is treated with a so-called equilibrium extract, resulting only in the extraction of caffeine from the coffee, followed by recovery of the caffeine from said caffeine-loaded equilibrium extract using activated carbon.

Various other extraction methods using activated carbon can also be used.

The treatment of the activated carbon loaded with caffeine can be carried out under conditions and in systems known in the art. After the recovery of the caffeine from the activated carbon the caffeine-loaded recovery agent solution can be treated to remove the caffeine therefrom. A suitable manner can be simply the evaporation of the recovery agent under conditions of temperature and pressure sufficient to evaporate it and condensation of the clean recovery agent. The recovered caffein may be purified further by recrystallization.

Preferably the active carbon is treated with steam after the removal of the caffeine from it, to remove any remaining solvent before re-use of the carbon.

According to the invention various recovery agents are used. All of them have the advantage that they more selectively desorb the caffeine from the activated carbon than the known agents like acetic acid. Especially dichloromethane has been found to remove the caffeine almost selectively from the activated carbon, without substantial removal of other soluble coffee solids from the carbon. The mixture of methylethylketone and ethyl acetate, in various ratios (1/10 to 10/1, preferably 1/2 to 2/1), preferably having a slight excess of ethyl acetate, is almost as selective as dichloromethane, but is somewhat less attractive from a point of view of energy economics. The same argument applies to ethylacetate, whereas methylethylketone turns out to be a bit more favorable on energy economics, but to have a rather higher loss of dry solids, although that is even a factor two better than acetic acid.

Especially suitable combinations of recovery agent and temperature are the following:

| Recovery agent | Temperature range |
|---|---|
| Dichloromethane | 20-80°C |
| MEK/EA* (40/60) | 50-100°C |
| MEK* | 50-100°C |
| EA* | 50-100°C |

| | |
|---|---|
| *: MEK is methyl ethyl ketone EA is ethyl acetate | |

The invention is now elucidated on the basis of some examples.

### EXAMPLES 1-5

In order to show the effectiveness of various recovery agents known quantities of an activated carbon having a speciific surface area of > 1000 m²/g and a specific pore volume of > 0.5 cm³/g, were treated with caffeine containing coffee extract, resulting in caffeine loaded activated carbon. After washing with water the caffeine loading of the carbon was determined.

Samples of the loaded activated carbon were treated with various recovery agents at a temperature just below the boiling point of the lowest boiling component of the recovery agent.

Three types of coffee were used in the experiments, however no significant difference could be found in the results between the various types of coffee. Therefor the experiments reported herein are based on the mean values of the experiments for all three types of coffee.

In the experiments the following recovery agents indicated in the table were used.

| Example | Recovery agent | Temperature °C |
|---|---|---|
| 1 | Dichloromethane | 35 |
| 2 | MEK/EA | 70 |
| 3 | MEK | 70 |
| 4 | EA | 70 |
| 5 | Acetic acid 100% | 100 |

The results of the experiments are given in the table below. The selectivity is defined as the percentage of caffeine present in the desorbed material.

| Example | Recovery agent | % caffeine in desorbed material % |
|---|---|---|
| 1 | Dichloromethane | 63 |
| 2 | MEK/EA | 55 |
| 3 | MEK | 37 |
| 4 | EA | 59 |
| 5 | Acetic acid 100% | 18 |

This table clearly shows the superior results of the present invention.

In case the total energy requirements of the process are also taken into account the economy of the process of the present invention becomes even more pronounced as follows from the following table. In this table the total costs resulting from removal of solvent and the loss of non-caffein dry solids have been given, using acetic acid as a reference (100%).

| Example | Recovery agent | Total costs % |
|---|---|---|
| 1 | Dichloromethane | 32 |
| 2 | MEK/EA | 56 |
| 3 | MEK | 62 |
| 4 | EA | 651 |
| 5 | Acetic acid 100% | 100 |

## Claims

1. Process for recovering caffeine from activated carbon loaded with caffeine, said process comprising contacting said activated carbon having caffeine adsorbed thereon with methyl-ethylketone, ethylacetate, dichloromethane, or a mixture of methyl-ethylketone and ethylacetate for a period of time sufficient to desorb at least a portion of the caffeine from the activated carbon.

2. Process according to claim 1, wherein the said caffeine loaded activated carbon has been obtained from decaffeinating vegetable matter, such such as coffee and tea.

3. Process according to claim 1 or 2, wherein the activated carbon having caffeine adsorbed thereon also contains water.

4. Process according to any one of the claims 1-3, wherein the activated carbon is contacted with the recovery agent at a temperature ranging from 20 to 90°C, preferably from 40 to 85°C.

5. Process according to any one of the claims 1-4, wherein the caffeine is recovered from the recovery agent in which it is dissolved by evaporation of the solvent.

6. Process according to claim 1-5, wherein the caffein is recrystalized after recovery from the solvent.

7. Process for decaffeinating green coffee, said process comprising extracting caffeine from said green coffee into an aqueous liquid, adsorbing the caffeine from said aqueous liquid on activated carbon, and contacting said activated carbon having caffeine adsorbed thereon with methyl-ethylketone, ethylacetate, dichloromethane, or a mixture of methyl-ethylketone and ethylacetate for a period of time sufficient to desorb at least a portion of the caffeine from the activated carbon.

8. Process according to claim 7, wherein said aqueous liquid constitutes a caffeine-free equilibrium green coffee extract.

9. Process according to claim 7, wherein said aqueous liquid constitutes water or a solution substantially free from soluble green coffee solids, resulting in a green coffee extract from which the caffeine is removed by contact with the activated carbon, following which the caffeine freed green coffee extract is returned to extracted green coffee.

10. Process according to claim 7-9, wherein the green coffee is subsequently roasted.

11. Process according to claim 7-10, wherein the activated carbon, after desorption of caffeine from it, is reused in adsorbing caffeine from the said aqueous extract.

## Patentansprüche

1. Verfahren zur Wiedergewinnung von Koffein aus mit Koffein beladener Aktivkohle, wobei das Verfahren In-Kontakt-Bringen der Aktivkohle mit darauf adsorbiertem Koffein mit Methylethylketon, Ethylacetat, Dichlormethan oder einem Gemisch von Methylethylketon und Ethylacetat während einer Zeit umfaßt, die zur Desorption von mindestens einem Teil des Koffeins von der Aktivkohle ausreicht.

2. Verfahren nach Anspruch 1, wobei die mit Koffein beladene Aktivkohle aus der Entkoffeinierung von pflanzlichem Material wie Kaffee und Tee erhalten wurde.

3. Verfahren nach Anspruch 1 oder 2, wobei die Aktivkohle mit darauf adsorbiertem Koffein auch Wasser enthält.

4. Verfahren nach Anspruch 1 bis 3, wobei die Aktivkohle bei einer Temperatur im Bereich von 20 bis 90°C, bevorzugt 40 bis 85°C in Kontakt mit dem Wiedergewinnungsmittel gebracht wird.

5. Verfahren nach einem der Ansprüche 1 bis 4, wobei das Koffein durch Verdampfen des Lösungsmittels wiedergewonnen wird, in dem es gelöst ist.

6. Verfahren nach Anspruch 1 bis 5, wobei das Koffein nach Wiedergewinnung aus dem Lösungsmittel umkristallisiert wird.

7. Verfahren zur Entkoffeinierung von grünem Kaffee, wobei das Verfahren Extrahieren von Koffein aus dem grünen Kaffee in eine wäßrige Flüssigkeit, Adsorbieren des Koffeins aus der wäßrigen Flüssigkeit auf Aktivkohle, und In-Kontakt-Bringen dieser Aktivkohle mit darauf adsorbiertem Koffein mit Methylethylketon, Ethylacetat, Dichlormethan oder einem Gemisch von Methylethylketon und Ethylacetat während einer Zeit umfaßt, die ausreichend ist zur Desorption von mindestens einem Teil des Koffeins von der Aktivkohle.

8. Verfahren nach Anspruch 7, wobei die wäßrige Flüssigkeit einen koffeinfreien, Gleichgewichts-Extrakt aus grünem Kaffee darstellt.

9. Verfahren nach Anspruch 7, wobei die wäßrige Flüssigkeit Wasser oder eine Lösung darstellt, die im wesentlichen frei ist von löslichen Feststoffen aus grünem Kaffee, resultierend in einem Extrakt aus grünem Kaffee, aus dem Koffein durch In-Kontakt-Bringen mit der Aktivkohle entfernt wird, dadurch gefolgt, daß der vom Koffein befreite Extrakt aus grünem Kaffee dem extrahierten grünen Kaffee wieder zugesetzt wird.

10. Verfahren nach Anspruch 7 bis 9, wobei der grüne Kaffee anschließend geröstet wird.

11. Verfahren nach Anspruch 7 bis 10, wobei die Aktivkohle nach Desorption des Koffeins bei der Adsorption von Koffein aus dem wäßrigen Extrakt wiederverwendet wird.

## Revendications

1. Procédé pour récupérer la caféine à partir de charbon actif chargé de caféine, ledit procédé consistant à mettre ledit charbon actif ayant de la caféine adsorbée sur celui-ci en contact avec de la méthyléthylcétone, de l'acétate d'éthyle, du dichlorométhane ou un mélange de méthyléthylcétone et d'acétate d'éthyle pendant une durée suffisante pour désorber au moins une portion de la caféine d'avec le charbon actif.

2. Procédé selon la revendication 1, dans lequel ledit charbon actif chargé de caféine a été obtenu en décaféinant une matière végétale, telle que du café et du thé.

3. Procédé selon la revendication 1 ou 2, dans lequel le charbon actif possédant de la caféine adsorbée sur celui-ci contient également de l'eau.

4. Procédé selon l'une quelconque des revendications 1 à 3, dans lequel le charbon actif est mis en contact avec l'agent de récupération à une température allant de 20 à 90°C, de préférence de 40 à 85°C.

5. Procédé selon l'une quelconque des revendications 1 à 4, dans lequel la caféine est récupérée à partir de l'agent de récupération dans lequel elle s'est dissoute par évaporation du solvant.

6. Procédé selon les revendications 1 à 5, dans lequel la caféine est recristallisée après récupération à partir du solvant.

7. Procédé pour décaféiner du café vert, ledit procédé consistant à extraire la caféine dudit café vert dans un liquide aqueux, à adsorber la caféine à partir dudit liquide aqueux sur du charbon actif et à mettre ledit charbon actif ayant de la caféine adsorbée sur celui-ci en contact avec de la méthyléthylcétone, de l'acétate d'éthyle, du dichlorométhane, ou un mélange de méthyléthylcétone et d'acétate d'éthyle pendant une durée suffisante pour désorber au moins une portion de la caféine d'avec le charbon actif.

8. Procédé selon la revendication 7, dans lequel ledit liquide aqueux est constitué d'un extrait de café vert d'équilibre exempt de caféine.

9. Procédé selon la revendication 7, dans lequel ledit liquide aqueux est constitué d'eau ou d'une solution essentiellement exempte de solides solubles du café vert, aboutissant à un extrait de café vert duquel la caféine est retirée par contact avec le charbon actif, à la suite de quoi l'extrait de café vert débarrassé de caféine est retourné au café vert extrait.

10. Procédé selon les revendications 7 à 9, dans lequel le café vert est torréfié par la suite.

11. Procédé selon les revendications 7 à 10, dans lequel le charbon actif, après désorption de la caféine de celui-ci, est réutilisé dans l'adsorption de la caféine à partir dudit extrait aqueux.
